Europäisches Patentamt

European Patent Office

Office européen des brevets

(19)

(11) Publication number: 0 256 415
A2

(12) **EUROPEAN PATENT APPLICATION**

(21) Application number: 87111221.5

(22) Date of filing: 04.08.87

(51) Int. Cl.⁴: **A61B 5/14 , G01N 1/14**

(30) Priority: 15.08.86 SE 8603428

(43) Date of publication of application:
24.02.88 Bulletin 88/08

(84) Designated Contracting States:
BE CH DE ES FR GB IT LI NL

(71) Applicant: GAMBRO AB
Post Box 10101
S-220 10 Lund(SE)

(72) Inventor: Claren, Jan Seivert
Protokollgränden 38
S-222 47 Lund(SV)
Inventor: Felding, Anders
Östra Förstadsgatan 21
S-211 31 Malmö(SV)

(74) Representative: Boberg, Nils Gunnar Erik
Gambro AB Patent Department Box 10101
S-220 10 Lund(SE)

(54) **System of analysis.**

(57) A system of analysis, comprising means for the withdrawal of blood from a patient or other source (1) and for the conducting of the same past a measuring electrode (5) or the like and means for the feed of a diluting and/or anticoagulant fluid (13) to a point near the said source (1).

The system is characterized in that the said means comprise one or more pumps (10, 14), with the help of which blood samples can be drawn intermittently, and which in between are adapted to feed some of the said fluid to the said point near the sampling point.

The system is intended in particular for the measurement of potassium, calcium and sodium in the blood, but may also be used for the measurement of other substances, e g glucose in connection with diabetes.

Fig. 1

EP 0 256 415 A2

## SYSTEM OF ANALYSIS

### TECHNICAL FIELD

The present invention relates to a system of analysis, comprising means for the withdrawal of blood from a patient or other source and for the conducting of the same past a measuring electrode or the like and means for the feed of a diluting and/or anticoagulant fluid to a point near the said source.

The system in accordance with the invention is intended in particular for the analysis of potassium, sodium and calcium in blood. It will be obvious, however, to those versed in the art that it may also be used for the analysis of many other substances, e g glucose in connection with diabetes.

### BACKGROUND ART

In the American patent specifications US 4 229 542, US 4 352 374, US 4 123 353, US 4 587 219, for example, different systems of analysis are described intended primarily for the continuous analysis of blood and other complicated fluids. These systems are less suitable, however, for intermittent sampling. Furthermore, these systems normally require the blood first to be dialysed, before tha analysis is carried out in the dialysate with the help of a measuring electrode or the like. For the rest, however, the systems described present substantial advantages.

### DISCLOSURE OF THE INVENTION

It is an object of the present invention, whilst making use of the advantages of the above mentioned systems, to provide a system of analysis of the type defined above which is also suitable for intermittent sampling, and where the blood does not have to be dialysed before the analysis. More particularly, with the help of the invention a system of analysis is provided which comprises means for the withdrawal of blood from a patient or other source and for the conducting of the same past a measuring electrode or the like and means for the feed of a diluting and/or anticoagulant fluid to a point near the said source. It is characteristic for this system that the said means comprise one or more pumps, with the help of which blood samples can be drawn intermittently, and which in between are adapted to feed some of the said fluid to a point near the sampling point.

This sampling point may consist, for example, or a double-lumen cannula arranged in the vein of a patient. Between the samplings this cannula is thus kept open with the help of the fluid fed to it. Preferably this is done by adapting the said pump or pumps to feed a smaller quantity of the fluid continuously to the said point between samplings. Alternatively, however, they may also be adapted, to feed fluid intermittently to the said point between samplings, and this in partular just before each sampling.

Preferably, moreover, the said means are suitably adapted to conduct the fluid from the said point near the source further to the measuring electrode and past this to a drain or the like. In this way the measuring electrode too is continuously or intermittently resned clean between samplings.

A preferred embodiment of the system of analysis in accordance with the invention is characterized by a valve arranged between the source and the electrode which is adapted to connect alternatively one or more sources of calibrating solutions for the conducting of these solutions past the measuring electrode. This valve may be designed, for example, in accordance with the description given in the afore mentioned American patent 4 587 219. An advantage gained through this design is that the above mentioned cannula can remain stuck into the patient even when the system is being calibrated.

It is appropriate for a check valve to be arranged between the said valve and the said source or sources of calibrating solutions. This check valve prevents any other fluids included in the system from being pressed into, or being mixed with, the calibrating solutions by mistake.

To ensure that the least possible quantity of blood is used up during each sampling, the said pump or pumps may be adapted immediately following each sampling to project forward the sample drawn towards the measuring electrode by pumping a suitable quantitiy of the said fluid after the same.

Appropriately a pump is also arranged after the measuring electrode for drawing the blood past the same. Should in the course of this a haemolysis of the blood take place, the measuring result will not be affected by this.

A pump is preferably arranged between the source of the said fluid and the valve for pressing this fluid via the valve past the said point near the source of the blood and further past the measuring electrode. As a result an effective flushing is obtained of the valve as well as of the sampling point and the measuring electrode.

Appropriately the two last mentioned pumps are adapted so that between the samplings they are driven at such speeds that smaller quantity of the fluid is pressed into the source of the blood. If this source consists of a double-lumen cannula arranged in a vein, this design ensures that this cannula is kept flushed out up to the tip, so that any coagulation in or around the same is avoided with certainty.

## BRIEF DESCRIPTION OF DRAWINGS

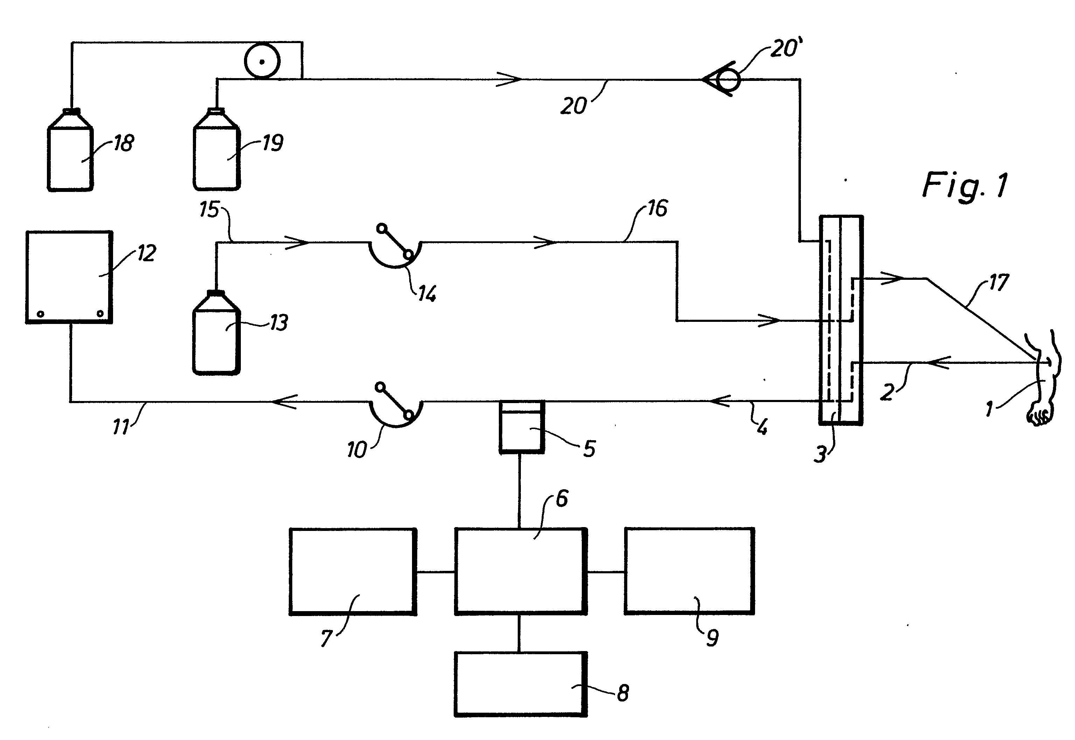

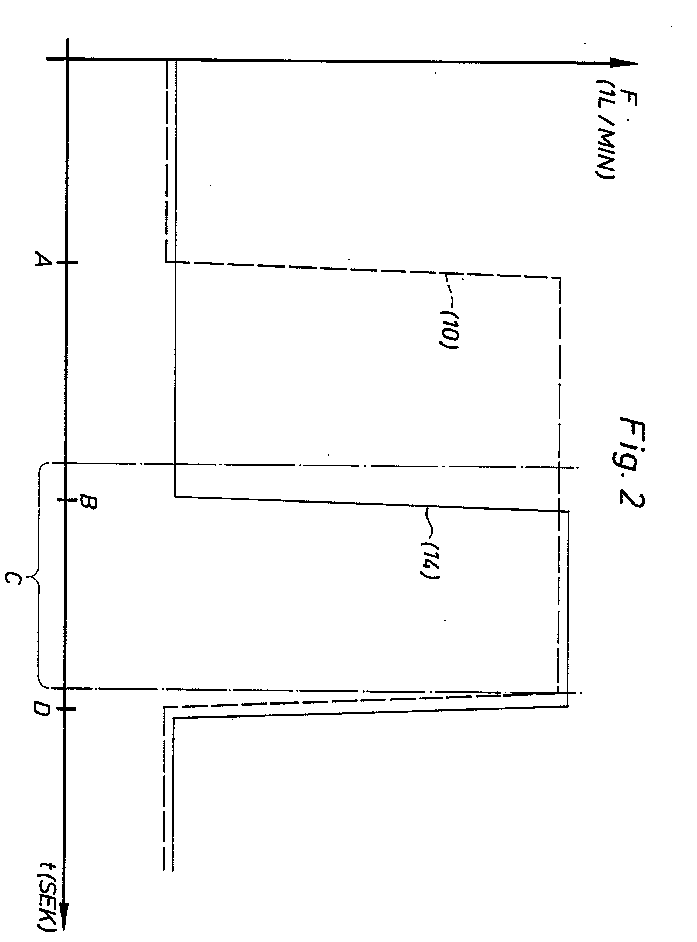

The invention is described in more detail in the following with reference to the attached drawings wherein Fig 1 shows a schematic diagram and Fig 2 a flow diagram.

## BEST MODE OF CARRYING OUT THE INVENTION

In Fig 1 is this shown a schematic diagram of a system of analysis carried out in accordance with the present invention. The patient or some other source of blood is marked 1. With the help of a duct 2 this blood is passed via a valve 3 and and a duct 4 to and electrode 5. The analysed result is introduced into a computer 6 and is read out with the help of a printer 7 or a display 8. Reference 9 indicates that the system may be connected to a second computer, e g monitor or a central computer. Alternatively the unit 9 may be considered to represent a control panel or the like for the feeding in of appropriate operating data.

A pump marked 10 draws blood from the source 1 via the electrode 5 and further through a duct 11 to a drain bag 12.

A source of a salt solution with or without anticoagulant is marked 13. This salt solution is intended to constitute a diluting and/or anticoagulant fluid which with the help of a pump 14 is pumped through ducts 15, 16 and 17 via the valve 3 to a point near the source 1 of the blood.

Two sources of different calibrating solutions are marked 18 and 19. These calibrating solutions can be drawn with the help of the pump 10 through a duct 20 with a check valve 20' and the ducts 4 and 11 past the electrode to the drain 12. During this calibration the patient 1 thus continues to be connected to the valve 3, but is no longer in communication with the duct 4 and the measuring electrode 5.

In Fig 2 is represented a flow diagram for the two pumps 10 and 14. Up to the point A is shown the situation between two samplings. The two pumps are then driven at low speed, e g at a speed of approx. 0,33 millilitre per minute. The pump 14 is suitable adapted so that it is driven at a slightly higher speed than the pump 10, which means that a small quantity of fluid form the source 13 is pressed into the patient 1. At the point A a sampling process is started in that the capacity of the pump 10 is increased, for example, to an order of magnitude of 1,5 millilitre per minute. This quantitity is mixed with a small quantity of diluting fluid which is delivered by the pump 14. At the point B the speed of the pump 14 too is increased with the result that the withdrawal of blood is stopped. Instead a corresponding quantity of fluid is delivered by the pump 14. At the point B the speed of the pump 14 too is increased with the result that the withdrawal of blood is stopped. Instead a corresponding quantity of fluid is delivered from the source 13 in such a manner that it projects the blood forward to the measuring electrode 5. The time the blood is in contact with the measuring electrode 5 is marked C. During this time a certain stabilization of the measured value takes place, so that the most accurately measured value is obtained towards the end of this measuring period. At the point D the sampling has been completed, so that the pumps can return to their lower capacity. For the sake of clarity this has been shown in the diagram with a certain time delay. Appropriately it ought to be possible, though, for the capacity of both the pumps to be lowered at substantially the same time.

The calibration appropriately proceeds in the same manner as the sampling. The only difference is that the valve 3 is changed over in such a manner that the pump 10 draws in calibration solution instead of blood.

Naturally the invention is not limited merely to the embodiment described above, but can be varied within the scope of the following claims. For example, the system in accordance with the invention can also be used for intermittent sampling with the help of a standard blood sample syringe. In such a case the valve 3 is designed in such a manner that the patient 1 can be disconnected, and it provided with an injection port or the like, so that the sample taken with the help of the sryinge can be injected directly into the blood duct in the valve.

## Claims

1. A system of analysis comprising means for the withdrawal of blood from a patient or other source (1) and for the conducting of the same past a measuring electrode (5) or the like and means for the feed fo a diluting and/or coagulant fluid (13) to a point near the said source (1), **characterized** in that the said means comprise one or more pumps (10, 14), with the help of which blood samples can be drawn intermittently, and which in between are adapted to feed some of the said fluid (13) to the said point near the source or the sampling point (1).

2. A system of analysis in accordance with claim 1, **characterized** in that the said pump or pumps (10, 14) are adapted to feed a smaller quantity of the fluid continuously to the said point between the samplings.

3. A system of analysis in accordance with claim 1, **characterized** in that the said pump or pumps (10, 14) are adapted to feed the fluid (13) intermittently to the said point between samplings and this in paricular just before each sampling.

4. A system of the analysis in accordance with the preceding claims, **characterized** in that the said means are adapted to conduct the fluid from the said point near the source (1) further to the measuring electrode (5) and past this to a drain or the like (12).

5. A system of analysis in accordance with anyone of the preceding claims, **characterized** by a valve (3) arranged between the source (1) and the measuring electrode (5) which is adapted to connect alternatively one or more sources (18, 19) of calibrating solutions for the conducting of these solutions past the measuring electrode (5).

6. A system of analysis in accordance with claim 5, **characterized** by a check valve (20') arranged between the valve (3) and the said source or sources (18, 19) of calibrating solutions.

7. A system of analysis in accordance with anyone of the preceding claims, **characterized** in that the said pump or pumps (10, 14) are adapted immediately following each sampling to project forward the sample drawn towards the measuring electrode (5) by pumping a suitable quantity of the said fluid (13) after the same.

8. A system of analysis in accordance with anyone of the preceding claims, **characterized** by a pump (10) arranged after the measuring electrode (5) for drawing the blood past the same.

9. A system of analysis in accordance with claim 5, **characterized** by a pump (14) arranged between the source (13) of the said fluid and the valve (3) for pressing this fluid via the valve past the said point near the source (1) of the blood and further past the measuring electrode (5).

10. A system of analysis in accordance with claim 8 and 9, **characterized** in that the said pumps (10, 14) are arranged so that between the samplings they are driven at such speeds that a smaller quantity of the fliud (13) is pressed into the source (1) of the blood.

Fig. 1

0 256 415

*Fig. 2*

F
(1L/MIN)

A

(10)

B

C

(14)

D

t (SEK)